(19) Europäisches Patentamt
European Patent Office
Office européen des brevets

(11) Numéro de publication : **0 346 250 B1**

(12) # FASCICULE DE BREVET EUROPEEN

(45) Date de publication du fascicule du brevet : **25.11.92 Bulletin 92/48**

(51) Int. Cl.$^5$ : **C07C 37/60,** C07C 41/26

(21) Numéro de dépôt : **89420188.8**

(22) Date de dépôt : **31.05.89**

(54) **Procédé d'hydroxylation de phénols et d'éthers de phénols.**

(30) Priorité : **08.06.88 FR 8807882**

(43) Date de publication de la demande : **13.12.89 Bulletin 89/50**

(45) Mention de la délivrance du brevet : **25.11.92 Bulletin 92/48**

(84) Etats contractants désignés : **AT BE CH DE ES FR GB GR IT LI LU NL SE**

(56) Documents cités :
**FR-A- 2 489 816**
**US-A- 3 580 956**
**US-A- 3 662 006**

(73) Titulaire : **RHONE POULENC CHIMIE
25 Quai Paul Doumer
F-92408 Courbevoie Cédex (FR)**

(72) Inventeur : **Costantini, Michel
10, rue du Docteur Bonhomme
F-69003 Lyon (FR)**
Inventeur : **Gubelmann, Michel
39, Boulevard des Belges
F-69006 Lyon (FR)**
Inventeur : **Lecomte, Jean-Pierre
24, rue Boileau
F-69006 Lyon (FR)**
Inventeur : **Popa, Jean-Michel
2, rue Roger Breton
F-93700 Drancy (FR)**

(74) Mandataire : **Dutruc-Rosset, Marie-Claude et al
RHONE-POULENC CHIMIE Direction des Brevets 25, Quai Paul Doumer
F-92408 Courbevoie Cédex (FR)**

EP 0 346 250 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé d'hydroxylation de phénols ou d'éthers de phénols par le peroxyde d'hydrogène.

L'hydroxylation du phénol ou de phénols substitués, par le peroxyde d'hydrogène, pour préparer des diphénols est une réaction connue.

Le brevet français n° 69-45467 publié sous le n° 2 071 464 a décrit un procédé dans lequel la réaction est catalysée par un acide fort, tel que par exemple l'acide perchlorique ou l'acide sulfurique.

Le brevet allemand n° 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, qui se forment le plus souvent lors de l'élimination des catalyseurs acides dissous.

Ainsi, la demande de brevet français n° 81-17023 (publiée sous le n° 2 489 816) préconise l'emploi de silicalite de titane comme catalyseur hétérogène de l'hydroxylation de composés aromatiques par le peroxyde d'hydrogène.

la fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n° 200 260, d'utiliser des agglomérats de ces fines particules de silicalite de titane.

Il s'avère cependant que l'on recherche encore une catalyse hétérogène de la réaction d'hydroxylation des phénols ou éthers de phénols par le peroxyde d'hydrogène, qui puisse être utilisée de manière industrielle dans des conditions économiquement acceptables. C'est précisément l'objet de la présente invention.

L'invention consiste donc en un procédé d'hydroxylation de phénols ou éthers de phénols de formule générale (I) :

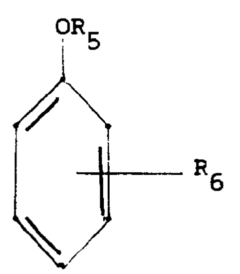

(I)

dans laquelle :

- $R_5$ représente un atome d'hydrogène, un groupement méthyle, un groupement éthyle ou un groupement phényle,
- $R_5$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace de zéolite de structure MFI à base d'oxyde de silicium et d'oxyde de germanium et ayant après calcination la formule suivante (II) :

$$Si_{(96-x)},\ Ge_x\ O_{192} \quad (II)$$

dans laquelle :

x est compris entre 0,1 et 36 environ.

Ces zéolites seront dénommées dans le présent texte germanozéosilites.

La germanozéosilite utilisée dans le procédé de l'invention a un système cristallin monoclinique et un diagramme de diffraction des rayons X défini dans le tableau I.

Dans ce tableau, les valeurs extrêmes des différentes équidistances réticulaires $d_{hkl}$ sont données et correspondent aux concentrations limites de germanium incorporé dans la charpente de la zéolite, ou plus précisément au rapport Ge/(Si + Ge).

En effet, l'identification des germanozéosilites peut être notamment et avantageusement réalisée par l'établissement de leur diagramme de diffraction des rayons X.

Ce diagramme de diffraction peut être obtenu à l'aide d'un diffractomètre en utilisant la méthode classique des poudres avec le rayonnement K$\alpha$ du cuivre. A partir de la position des pics de diffraction représentée par l'angle 2$\theta$ on calcule, par la relation de Bragg, les équidistances réticulaires $d_{hkl}$ caractéristiques de l'échantillon. L'estimation de l'erreur de mesure $\Delta$ ($d_{hkl}$) sur $d_{hkl}$ se calcule, en fonction de l'erreur absolue $\Delta$ (2$\theta$) affectée à la mesure de 2$\theta$, par la relation de Bragg. Une erreur absolue $\Delta$ (2$\theta$) égale à ± 0,2° est couramment admise. L'intensité relative $I/I_o$ affectée à chaque valeur de $d_{hkl}$ est estimée à partir de la hauteur du pic de diffraction correspondant. On utilise souvent une échelle de symboles pour caractériser cette intensité : FF = très fort, F = fort, mF = moyen à fort, m = moyen, mf = moyen à faible, f = faible, ff = très faible.

La valeur du volume $V_o$ de la maille cristallographique des germanozéosilites est fonction de la substitution du silicium par le germanium.

Selon une autre caractéristique, les germanozéosilites utilisées dans l'invention peuvent contenir du fluor lorsque les anions $F^-$ ont été utilisés comme mobilisateur. La concentration en fluor est généralement comprise entre 0,01 et 1,4 % en poids après calcination. Ce fluor peut toutefois être éliminé par un traitement hydrothermal à pH > 7 sans pour cela modifier la structure de la germanozéosilite de l'invention.

TABLEAU I

diagramme de diffraction des rayons X

| Valeurs extrêmes des $d_{hkl}$ (nm) | $I/I_o$ | Valeurs extrêmes des $d_{hkl}$ (nm) | $I/I_o$ |
|---|---|---|---|
| 1,120-1,123 | F-FF | 0,460 -0,464 | f |
| 0,992-1,004 | F-FF | 0,444 -0,449 | f |
| 0,973-0,984 | f | 0,434 -0,439 | f |
| 0,896-0,904 | ff | 0,422 -0,427 | f |
| 0,803-0,811 | ff | 0,406 -0,411 | ff |
| 0,742-0,750 | ff (large) | 0,400 -0,403 | f |
| 0,705-0,712 | ff | 0,3835-0,3875 | F |
| 0,667-0,676 | f | 0,3805-0,3845 | mF |
| 0,633-0,640 | f | 0,3780-0,3820 | mF |
| 0,595-0,602 | mf | 0,3740-0,3780 | m |
| 0,590-0,596 | f | 0,3715-0,3755 | m |
| 0,570-0,576 | mf | 0,3710-0,3750 | m |
| 0,566-0,572 | f (épaulement) | 0,3650-0,3690 | f |
| 0,555-0,564 | f | 0,3615-0,3650 | f |
| 0,535-0,541 | f | 0,3480-0,3515 | ff (large) |
| 0,532-0,538 | f | 0,3435-0,3470 | f |
| 0,511-0,516 | ff (large) | 0,3415-0,3450 | f |
| 0,502-0,506 | ff | 0,3385-0,3422 | ff |
| 0,496-0,501 | mf | 0,3342-0,3377 | f (large) |
| 0,486-0,491 | ff (large) | 0,3295-0,3329 | f |
| 0,469-0,474 | ff | 0,3245-0,3279 | f |

La germanozéosilite servant de catalyseur dans le présent procédé peut être synthétisée de la manière suivante :

(i) préparation d'un mélange réactionnel en milieu aqueux contenant au moins une source de silicium au degré d'oxydation + 4, une source de germanium au degré d'oxydation + 4, et un agent structurant,

(ii) cristallisation de ce mélange réactionnel par chauffage et récupération du précipité cristallisé, et

(iii) calcination de celui-ci à une température supérieure à 450°C pour l'élimination du structurant occlus dans les canaux.

L'agent mobilisateur est, pour un pH supérieur à environ 12, les ions $OH^-$. Pour un pH inférieur ou égal à environ 12, des ions $F^-$ sont ajoutés comme agent mobilisateur.

D'une façon générale, il est conseillé d'éviter la présence, dans le milieu réactionnel, de cations alcalins ou ammonium ($NH_4^+$) qui forment des composés insolubles de germanium tels que, par exemple, $KH_3Ge_2O_6$, $NH_4H_3Ge_2O_6$, $Na_2Ge_3O_77H_2O$, $K_3HGe_7O_{16}xH_2O$, $K_4Ge_9O_{20}$ et qui bloquent le germanium, empêchant ou limitant son incorporation dans la charpente de la germanozéosilite.

De nombreuses sources de l'élément silicium au degré d'oxydation +4 peuvent être utilisées. On peut citer à titre d'exemple, les silices sous forme d'hydrogels, d'aérogels, de xérogel, de suspensions colloïdales, les silices résultant de la précipitation à partir de solutions de silicates solubles ou de l'hydrolyse d'esters siliciques comme $Si(OC_2H_5)_4$, les silices préparées par des traitements d'extraction et d'activation de composés cristallisés ou amorphes naturels ou synthétiques comme les silicates d'aluminium, les aluminosilicates, les argiles. On peut également utiliser des composés du silicium tétravalent hydrolysables tels les halogénures de silicium ou analogues.

Parmi les sources de germanium au degré d'oxydation +4 on peut citer, à titre d'exemple, l'oxyde $GeO_2$ du type quartz, les composés du germanium pouvant être hydrolysés tels les alkoxydes, les halogénures ou analogues.

Il est également possible d'utiliser des composés contenant les éléments silicium et germanium associés tels que par exemple des verres ou des gels mixtes.

Les sources des éléments silicium et germanium au degré d'oxydation +4 peuvent être engagées sous forme de solutions ou de solides pulvérulents, mais également sous forme d'agglomérats, tels que, par exemple, pastilles ou extrudés, pouvant être transformés en germanozéosilite sans modification de la forme.

L'agent mobilisateur $OH^-$ est introduit sous forme de base(s) faibles(s) et/ou forte(s) ne contenant de préférence pas de cations alcalins ou $NH_4^+$. On peut citer les amines et les hydroxydes d'ammonium quaternaire.

L'agent mobilisateur $F^-$ est introduit sous forme d'acide et/ou de sel(s), ne contenant pas de cations alcalins ou $NH_4^+$, et/ou de composés libérant $F^-$ par hydrolyse. On peut citer, à titre d'exemple, l'acide fluorhydrique, les fluorhydrates d'amines, les fluorures d'ammonium quaternaire, $SiF_4$, $GeF_4$.

Les agents structurants convenables pour l'invention sont :

- les amines de formule (III) :

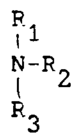

(III)

dans laquelle :

$R_1$, $R_2$, $R_3$ identiques ou différents représentent un groupe alkyle, de préférence un groupe propyle ou butyle ;

- les alkyles d'ammonium quaternaire de formule (IV)

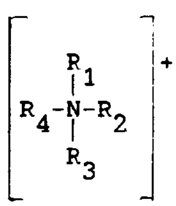

(IV)

dans laquelle :

$R_1$, $R_2$, $R_3$, $R_4$ identiques ou différents représentent des groupes alkyles, de préférence des groupes propyles ou butyles ;

- les composés de formules (III) et (IV) dans lesquelles l'azote a été remplacé par un atome de phosphore.

De préférence les agents structurants sont les composés qui peuvent fournir des cations tétrapropylammonium ou tripropylammonium.

Le structurant pourra être introduit sous forme de base ou de sel selon la nature du ou des mobilisateur(s) choisi(s) qui va déterminer le domaine de pH du milieu réactionnel.

Ainsi, en absence d'anion $F^-$, le pH élevé nécessaire à la synthèse pourra être obtenu par l'introduction du structurant sous forme d'hydroxyde d'ammonium quaternaire de formule (IV). Par contre, en présence d'anions $F^-$, le structurant pourra être introduit sous forme de sel d'ammonium quaternaire de formule (IV) ou de l'amine de formule (III), le pH étant éventuellement ajusté à l'aide d'une base. Avantageusement, cette base aura des propriétés d'agent structurant faible pour ne pas concurrencer l'agent structurant ajouté. Ainsi, les bases convenables pour l'invention sont à titre d'exemple, la méthylamine, la diméthylamine, la triméthylamine,

l'éthylamine, la diéthylamine et la triéthylamine.

Le mélange réactionnel a la composition suivante, exprimée en rapport molaire :

Ge/(Si+Ge) compris entre 0,001 et 0,80 ; de préférence, quand le pH est supérieur à 12 entre 0,002 et 0,8 et avantageusement entre 0,01 et 0,7 ; quand le pH est inférieur ou égal à 12 entre 0,001 et 0,75 et avantageusement entre 0,002 et 0,60.

Agent structurant/(Si+Ge) compris entre 0,002 et 4, de préférence entre 0,06 et 2 pour un pH supérieur à 12 et entre 0,06 et 1 pour un pH inférieur ou égal à 12.

F/(Si+Ge) compris entre 0,04 et 4, de préférence entre 0,06 et 2 pour un pH inférieur ou égal à 12.

$H_2O$/(Si+Ge) compris entre 4 et 400, de préférence 10 et 200 pour un pH supérieur à 12, et entre 20 et 200 pour un pH inférieur ou égal à 12.

Quand une base est utilisée pour ajuster le pH, le rapport molaire de la base par rapport à (Ge+Si) est compris entre 0 et 12, de préférence entre 2 et 8.

L'ajout à ce mélange réactionnel, de germes cristallisés de structure déterminée, par exemple MFI, dans une proportion qui n'excède pas quelques pourcents pondéraux par rapport au poids de $SiO_2$ + $GeO_2$ engagé peut faciliter la cristallisation de la germanozéosilite.

La cristallisation de la germanozéosilite peut être obtenue par chauffage du mélange réactionnel à une température comprise entre 40°C environ et 240°C environ, de préférence entre 60°C et 220°C pendant le temps nécessaire à la cristallisation, selon un mode opératoire classique de synthèse de zéolite et connu de l'homme du métier. A titre indicatif, la durée de chauffage peut être comprise entre 6 heures et 500 heures environ.

Ce chauffage et cette cristallisation sont réalisés de préférence dans un récipient ou autoclave revêtu d'une couche telle que, par exemple, le polytétrafluoroéthylène.

Le mélange réactionnel peut être agité ou non.

Après cristallisation, le précipité obtenu est recueilli, par exemple, par filtration.

Ce précipité est ensuite chauffé après un séchage éventuel, à une température supérieure à 450°C, de préférence supérieure à 500°C, afin de décomposer par calcination ou décomposition thermique les espèces organiques contenues dans le précipité, telles que, par exemple, l'agent structurant.

Les phénols et éthers de phénols qui sont utilisés de préférence dans le procédé de l'invention sont les composés de formule (I) dans laquelle $R_5$ représente un atome d'hydrogène, un groupement méthyle ou un groupement éthyle, et $R_6$ représente un atome d'hydrogène, un groupement méthyle, éthyle ou tertiobutyle, un groupement méthoxy ou éthoxy.

A titre d'exemple non limitatifs on peut citer le phénol, l'anisole, l'orthocrésol, le métacrésol, le paracrésol, le tertiobutyl-4 phénol, le méthoxy-2 phénol, le méthoxy-4 phénol.

Le procédé selon l'invention s'applique particulièrement bien au phénol pour la préparation d'hydroquinone et de pyrocatéchine.

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvant organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl-tertiobutyléther.

Le rapport molaire composé de formule I/peroxyde d'hydrogène est généralement de 25/1 à 3/1 et préférentiellement de 20/1 à 4/1.

La quantité de germanozéosilite définie précédemment, que l'on peut mettre en oeuvre dans le présent procédé peut varier dans de très larges limites.

Lorsque l'on réalise le procédé en discontinu, le catalyseur peut représenter en poids par rapport au poids du composé de formule (I) engagé de 0,1 % à 20 %. De préférence, ce rapport pondéral sera compris entre 0,5 % et 10%. Cependant si l'on réalise le procédé en continu, par exemple en faisant réagir un mélange de composé (I) et de solution de peroxyde d'hydrogène sur un lit fixe de catalyseur, ces rapports catalyseur/composé (I) n'ont plus de sens et, à un instant donné, on pourra avoir un excès pondéral de catalyseur par rapport au composé (I).

Il est également possible d'effectuer la réaction d'hydroxylation du composé (I) dans un solvant du composé (I), qui soit de préférence miscible ou partiellement miscible à l'eau.

A titre d'exemples non limitatifs de tels solvants, on peut citer l'eau ; les alcools comme le méthanol, le tertiobutanol, l'isopropanol ou l'éthanol ; les cétones comme l'acétone ou la méthylisobutylcétone ; les nitriles comme l'acétonitrile ; les acides carboxyliques comme l'acide acétique ; les esters comme l'acétate de propyle ; les éthers comme le méthyl-tertiobutyléther ; des solvants aprotiques polaires comme le dioxyde de tétrahydrothiophène (sulfolane), le carbonate d'éthylène glycol, le carbonate de propylène glycol, la N-méthylpyrrolidone.

La température à laquelle est conduite la réaction est généralement comprise entre 45° et 160°C sous pression atmosphérique. On peut également opérer à plus haute température et sous pression supérieure à la pression atmosphérique.

Les exemples qui suivent illustrent l'invention.

EXEMPLE 1 : PREPARATION DES CATALYSEURS 1a et 1b

Catalyseur 1a

Cet exemple décrit une synthèse d'une zéolite en milieu acide, avec utilisation des ions $F^-$ comme agent mobilisateur.

On prépare une solution A et un xérogel.

Solution A :

Elle est obtenue par mélange de tri-n-propylamine, de HF et de bromure de tétrapropylammonium (TPA-Br) : 4 g de HF à 50 % dans l'eau sont dilués dans 25 cm3 d'eau ; on ajoute 7,15 g de tri-n-propylamine sous agitation ; on poursuit l'agitation jusqu'à obtention d'une seule phase liquide ; on ajoute alors 6,65 g de TPA-Br dissous dans 27 cm3 d'eau.

Xérogel :

On mélange sous agitation 17 g de $SiCl_4$ et 2,15 g de $GeCl_4$ dans 20 cm3 de n.propanol, puis on ajoute 60 g d'eau goutte à goutte. On obtient un gel qui est séché à 80°C, jusqu'à obtention d'un poids de produit égal à 10,4 g.

On disperse lentement ce gel dans la solution A sous agitation.

Ce mélange a la composition suivante (ramenée à 1 mole de $SiO_2$) : 0,25 TPA-Br ; 0,5 Tri-n-propylamine ; 1 HF ; 1 $SiCl_4$ ; 0,1 $GeCl_4$ ; 30 $H_2O$.

On disperse dans ce mélange 0,12 g de cristaux broyés ayant une structure type MFI, comme germe de cristallisation. Le mélange réactionnel, caractérisé par un pH de 1,5, est chauffé 15 jours à 96°C. Après séparation des eaux mères, lavage abondant à l'eau et séchage à l'étuve à 80°C, on obtient 4,5 g de cristaux, contenant quelques particules amorphes, qui sont calcinés 6 heures sous air à 550°C.

Le diagramme de diffraction des rayons X obtenu sur le produit calciné est conforme aux valeurs du tableau I. La germanozéosilite obtenue a pour formule :

$$(Si_{91,3}\ Ge_{4,7})O_{192}$$

et elle contient en outre 0,6 % (pondérale) de Fluor.

Catalyseur 1b

On prépare tout d'abord une solution A et une solution B.

Solution A :

On verse goutte à goutte 6,432 g de $GeCl_4$ dans 1,4 cm3 d'une solution de HF à 50 % dans l'eau.

Solution B :

On dissout 5,362 g de TPA-Br dans 36 g d'eau, puis on coule 21,742 g d'une solution de $CH_3NH_2$ à 40 % dans l'eau.

On coule goutte à goutte la solution B sous agitation dans la solution A.

Il se forme un précipité blanc. On poursuit l'agitation pendant 15 minutes.

On disperse dans ce mélange 0,060 g de cristaux très finement broyés ayant une structure type MFI, comme germes de cristallisation, ainsi que 3,00 g de $SiO_2$ (sous forme d'aérosil).

On agite pendant 30 minutes.

Le pH du mélange est de 12 - 12,5.

Ce mélange a la composition suivante 1,25 $SiO_2$ ; 0,5 TPA-Br ; 7 $CH_3NH_2$ ; 1 HF ; 0,75 $GeCl_4$ ; 50 $H_2O$.
On transvase dans un récipient cylindrique de 100 cm3 en téflon, placé dans un autoclave métallique.
Après fermeture l'ensemble est chauffé pendant 15 heurs à 180 -190°C.
Après réaction et refroidissement, le pH est de 12.
On filtre, lave abondamment à l'eau et sèche à l'étuve à 80°C. On obtient une masse totale d'environ 4,3 g, contenant quelques particules amorphes, qui est calcinée 6 heures sous air à 550°C.
Le diagramme de diffraction des rayons X obtenu sur le produit calciné est conforme aux valeurs du tableau I. La germanozéosilite obtenue a pour formule :

$$(Si_{81}\ Ge_{15})O_{192}$$

EXEMPLE 2

Dans un réacteur en verre pyrex de 30 $cm^3$, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :
- 4,7 g de phénol (0,05 mol)
- 0,25 g de germanozéosilite préparée dans l'exemple 1a.
- 4,8 g d'eau distillée.

On chauffe à 100°C sous agitation, puis on injecte une solution aqueuse de $H_2O_2$ à 70 % en poids par volume. (0,0025 mol de $H_2O_2$).
On laisse ensuite réagir pendant 2 heures 30 minutes.
Après filtration du catalyseur, on dose $H_2O_2$ non transformée par iodométrie et les diphénols par chromatographie liquide haute performance (CLHP).
On obtient les résultats suivants :

| | |
|---|---|
| - taux de transformation (TT)de $H_2O_2$ : | 97,0 % |
| - rendement en pyrocatéchine par rapport à $H_2O_2$ transformée (RT) : | 35,0 % |
| - rendement en hydroquinone par rapport à $H_2O_2$ transformée (RT) : | 24,5 % |
| - rendement total en diphénols : | 59,5 % |

EXEMPLE 3

On répète l'exemple 2 avec les mêmes quantités de réactifs et dans les mêmes conditions opératoires, mais avec la germanozéosilite préparée dans l'exemple 1b.
On obtient les résultats suivants :

| | |
|---|---|
| - taux de transformation (TT)de $H_2O_2$ : | 93,0 % |
| - rendement en pyrocatéchine par rapport à $H_2O_2$ transformée (RT) : | 13,5 % |
| - rendement en hydroquinone par rapport à $H_2O_2$ transformée (RT) : | 9,0 % |
| - rendement total en diphénols : | 22,5 % |

**Revendications**

1. Procédé d'hydroxylation de phénols ou éthers de phénols de formule générale (I) :

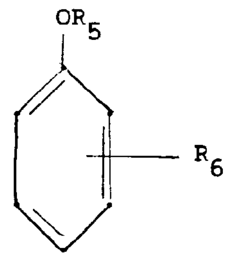

(I)

dans laquelle :

- $R_5$ représente un atome d'hydrogène, un groupement méthyle, un groupement éthyle ou un groupement phényle,
- $R_5$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace de zéolite de structure MFI à base d'oxyde de silicium et d'oxyde de germanium et ayant après calcination la formule suivante (II) :

$$Si_{(96-x)}, Ge_x O_{192} \quad (II)$$

dans laquelle :

x est compris entre 0,1 et 36 environ.

2. Procédé selon la revendication 1, caractérisé en ce qu'il est appliqué aux phénols et éthers de phénol de formule (I) dans laquelle $R_5$ représente un atome d'hydrogène, un groupement méthyle ou un groupement éthyle, et $R_5$ représente un atome d'hydrogène, un groupement méthyle, éthyle ou tertiobutyle, un groupement méthoxy ou éthoxy.

3. Procédé selon l'une des revendications 1 ou 2 caractérisé en ce qu'il est appliqué aux phénols et éthers de phénols choisi parmi le phénol, l'anisole, l'orthocrésol, le métacrésol, le paracrésol, le tertiobutyl-4 phénol, le méthoxy-2 phénol, le méthoxy-4 phénol.

4. Procédé selon l'une des revendications 1 à 3, caractérisé en ce que la germanozéosilite contient entre 0,01 et 1,4 % de fluor en poids après calcination.

5. Procédé selon l'une des revendications 1 à 4, caractérisé en ce que la germanozéosilite utilisée est obtenue par un procédé comportant les étapes suivantes :
   (i) préparation d'un mélange réactionnel en milieu aqueux contenant au moins une source de silicium au degré d'oxydation + 4, une source de germanium au degré d'oxydation + 4, et un agent structurant,
   (ii) cristallisation de ce mélange réactionnel par chauffage et récupération du précipité cristallisé, et
   (iii) calcination de celui-ci à une température supérieure à 450°C pour l'élimination du structurant occlus dans les canaux.

6. Procédé selon la revendication 5, caractérisé en ce que lors de la préparation de la germanozéosilite le rapport molaire Ge/(Si + Ge) dans le mélange réactionnel est compris entre 0,001 et 0,80 et de préférence entre 0,01 et 0,70 quand le pH est supérieur à 12.

7. Procédé selon la revendication 5, caractérisé en ce que lors de la préparation de la germanozéosilite le rapport molaire Ge/(Si + Ge) dans le mélange réactionnel est compris entre 0,001 et 0,75 et de préférence entre 0,002 et 0,60 quand le pH est inférieure ou égal à 12.

8. Procédé selon l'une des revendications 5 et 7, caractérisé en ce que lors de la préparation de la germanozéosilite le rapport molaire F/(Si + Ge) dans le mélange réactionnel est compris entre 0,06 et 2 pour un pH inférieur ou égal à 12.

9. Procédé selon l'une des revendications 5 et 6, caractérisé en ce que lors de la préparation de la germanozéosilite le rapport molaire $H_2O$/(Si + Ge) dans le mélange réactionnel est compris entre 4 et 400 et de préférence entre 10 et 200, pour un pH supérieur à 12.

10. Procédé selon l'une des revendications 5, 7 et 8, caractérisé en ce que lors de la préparation de la ger-

manozéosilite le rapport molaire $H_2O/(Si + Ge)$ dans le mélange réactionnel est compris entre 4 à 400 et de préférence entre 20 et 200, pour un pH inférieur ou égal à 12.

**11.** Procédé selon l'une des revendications 5, 6 et 9, caractérisé en ce que lors de la préparation de la germanozéosilite le rapport molaire agent structurant/(Si + Ge) est compris entre 0,002 et 4 et de préférence entre 0,06 et 2 pour un pH supérieur à 12.

**12.** Procédé selon l'une des revendications 5, 7, 8 et 10, caractérisé en ce que lors de la préparation de la germanozéosilite le rapport molaire agent structurant/(Si + Ge) est compris entre 0,002 et 4 et de préférence entre 0,06 et 1 pour un pH inférieur ou égal à 12.

**13.** Procédé selon l'une des revendications 1 à 12, caractérisé en ce que le rapport molaire phénol/peroxyde d'hydrogène est de 25/1 à 3/1 et de préférence de 20/1 à 4/1.

**14.** Procédé selon l'une des revendications 1 à 13 réalisé en discontinu, caractérisé en ce que le catalyseur représente en poids par rapport au poids du compose de formule (I) engagé de 0,1 % à 20 % et de préférence de 0,5 % à 10 %.

**15.** Procédé selon l'une des revendications 1 à 13, caractérisé en ce que le procédé est réalisé en continu sur un lit fixe de catalyseur.

**16.** Procédé selon l'une des revendications 1 à 15, caractérisé en ce que le peroxyde d'hydrogène est mis en oeuvre sous la forme d'une solution aqueuse.

**17.** Procédé selon l'une des revendications 1 à 15, caractérisé en ce que le peroxyde d'hydrogène est mis en oeuvre sous la forme d'une solution organique.

**18.** Procédé selon l'une des revendications 1 à 17, caractérisé en ce que la réaction d'hydroxylation est effectuée dans un solvant du composé de formule (I) de préférence miscible ou partiellement miscible à l'eau, tel que l'eau, un alcool, une cétone, un nitrile, un acide carboxylique, un ester, un éther ou un solvant aprotique polaire.

**19.** Procédé selon l'une des revendications 1 à 18, caractérisé en ce que la réaction d'hydroxylation est conduite à une température comprise entre 45°C et 160°C.

## Patentansprüche

**1.** Verfahren zur Hydroxylierung von Phenolen oder Phenolethern der allgemeinen Formel (I):

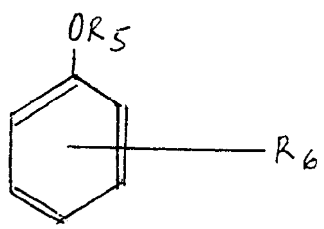

(I)

worin:

- $R_5$ ein Wasserstoffatom, eine Methylgruppe, eine Ethylgruppe oder eine Phenylgruppe bedeutet,
- $R_5$ ein Wasserstoffatom, einen Alkylrest mit 1 bis 4 Kohlenstoffatomen, einen Alkoxyrest mit 1 bis 4 Kohlenstoffatomen, einen Phenyl- oder Cyclohexylrest darstellt;

durch Reaktion mit Wasserstoffperoxid, dadurch gekennzeichnet, daß die Reaktion in Gegenwart einer wirksamen Zeolithmenge der Struktur MFI auf Basis von Siliziumoxid und Germaniumoxid durchgeführt wird, die nach Calcinierung die folgende Formel (II) hat:

$$Si_{(96-x)}, Ge_x O_{192} \quad (II)$$

worin:

x zwischen etwa 0,1 und 36 liegt.

2. Verfahren gemäß Anspruch 1, dadurch gekennzeichnet, daß es auf Phenole und Phenolether der Formel (I) angewandt wird, worin $R_5$ ein Wasserstoffatom, eine Methyl- oder Ethylgruppe bedeutet, und $R_6$ ein Wasserstoffatom, eine Methyl-, Ethyl- oder tert.-Butylgruppe, eine Methoxy- oder Ethoxygruppe darstellt.

3. Verfahren gemäß einem der Ansprüche 1 oder 2, dadurch gekennzeichnet, daß es auf Phenole und Phenolether angewandt wird, ausgewählt unter Phenol, Anisol, Orthokresol, Metakresol, Parakresol, 4-tert.-Butylphenol, 2-Methoxyphenol, 4-Methoxyphenol.

4. Verfahren gemäß einem der Ansprüche 1 bis 3, dadurch gekennzeichnet, daß das Germanozeosilit zwischen 0,01 und 1,4 Gewichtsprozent Fluor nach der Calcinierung enthält.

5. Verfahren gemäß einem der Ansprüche 1 bis 4, dadurch gekennzeichnet, daß das verwendete Germanozeosilit erhalten wird durch ein Verfahren mit den folgenden Stufen:
   (i) Herstellung eines Reaktionsgemisches in wäßrigem Milieu, das mindestens eine Siliziumquelle mit dem Oxidationsgrad + 4, eine Germaniumquelle mit dem Oxidationsgrad + 4, und ein strukturierendes Mittel enthält,
   (ii) Kristallisation dieses Reaktionsgemisches durch Erhitzen und Gewinnung des kristallisierten Niederschlags, und
   (iii) Calcinierung desselben bei einer Temperatur höher als 450 °C zur Entfernung des strukturierenden Mittels, das in den Kanälen eingeschlossen ist.

6. Verfahren gemäß Anspruch 5, dadurch gekennzeichnet, daß während der Herstellung des Germanozeosilits das Molverhältnis Ge/(Si + Ge) in dem Reaktionsgemisch zwischen 0,001 und 0,80 und vorzugsweise zwischen 0,01 und 0,70 liegt, wenn das pH höher als 12 ist.

7. Verfahren gemäß Anspruch 5, dadurch gekennzeichnet, daß während der Herstellung des Germanozeosilits das Molverhältnis Ge/(Si + Ge) in dem Reaktionsgemisch zwischen 0,001 und 0,75 und vorzugsweise zwischen 0,002 und 0,60 liegt, wenn das pH unter oder gleich 12 ist.

8. Verfahren gemäß einem der Ansprüche 5 und 7, dadurch gekennzeichnet, daß während der Herstellung des Germanozeosilits das Molverhältnis F/(Si + Ge) in dem Reaktionsgemisch zwischen 0,06 und 2 für ein pH unter oder gleich 12 liegt.

9. Verfahren gemäß einem der Ansprüche 5 und 6, dadurch gekennzeichnet, daß während der Herstellung des Germanozeosilits das Molverhältnis $H_2O$/(Si + Ge) in dem Reaktionsgemisch zwischen 4 und 400 und vorzugsweise zwischen 10 und 200 für ein pH über 12 liegt.

10. Verfahren gemäß einem der Ansprüche 5, 7 und 8, dadurch gekennzeichnet, daß während der Herstellung des Germanozeosilits das Molverhältnis $H_2O$/(Si + Ge) in dem Reaktionsgemisch zwischen 4 und 400 und vorzugsweise zwischen 20 und 200 für ein pH unter oder gleich 12 liegt.

11. Verfahren gemäß einem der Ansprüche 5, 6 und 9, dadurch gekennzeichnet, daß während der Herstellung des Germanozeosilits das Molverhältnis Strukturmittel/(Si + Ge) zwischen 0,002 und 4 und vorzugsweise zwischen 0,06 und 2 für ein pH über 12 liegt.

12. Verfahren gemäß einem der Ansprüche 5, 7, 8 und 10, dadurch gekennzeichnet, daß während der Herstellung des Germanozeosilits das Molverhältnis Strukturmittel/(Si + Ge) zwischen 0,002 und 4 und vorzugsweise zwischen 0,06 und 1 für ein pH unter oder gleich 12 liegt.

13. Verfahren gemäß einem der Ansprüche 1 bis 12, dadurch gekennzeichnet, daß das Molverhältnis Phenol/Wasserstoffperoxid 25/1 bis 3/1 und vorzugsweise 20/1 bis 4/1 beträgt.

14. Verfahren gemäß einem der Ansprüche 1 bis 13, diskontinuierlich durchgeführt, dadurch gekennzeichnet, daß der Katalysator gewichtsmäßig in bezug auf das Gewicht der eingesetzten Verbindung der Formel (I) von 0,1 % bis 20 % und vorzugsweise von 0,5 % bis 10 % beträgt.

15. Verfahren gemäß einem der Ansprüche 1 bis 13, dadurch gekennzeichnet, daß das Verfahren kontinuierlich auf einem festen Katalysatorbett durchgeführt wird.

16. Verfahren gemäß einem der Ansprüche 1 bis 15, dadurch gekennzeichnet, daß das Wasserstoffperoxid

in Form einer wäßrigen Lösung eingesetzt wird.

**17.** Verfahren gemäß einem der Ansprüche 1 bis 15, dadurch gekennzeichnet, daß das Wasserstoffperoxid in Form einer organischen Lösung eingesetzt wird.

**18.** Verfahren gemäß einem der Ansprüche 1 bis 17, dadurch gekennzeichnet, daß die Hydroxylierungsreaktion in einem Lösungsmittel der Verbindung der Formel (I), vorzugsweise mischbar oder teilweise mischbar mit Wasser, durchgeführt wird, wie Wasser, ein Alkohol, ein Keton, ein Nitril, eine Carbonsäure, ein Ester, ein Ether oder ein polares aprotisches Lösungsmittel.

**19.** Verfahren gemäß einem der Ansprüche 1 bis 18, dadurch gekennzeichnet, daß die Hydroxylierungsreaktion bei einer Temperatur zwischen 45 °C und 160 °C durchgeführt wird.

## Claims

**1.** Process for the hydroxylation of phenols or phenol ethers of general formula (I):

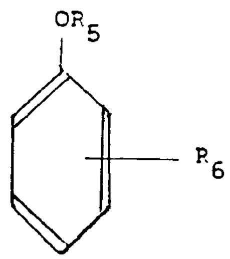

(I)

in which:

- $R_5$ denotes a hydrogen atom, a methyl group, an ethyl group or a phenyl group,
- $R_5$ denotes a hydrogen atom, an alkyl radical containing 1 to 4 carbon atoms, an alkoxy radical containing 1 to 4 carbon atoms, or a phenyl or cyclohexyl radical;

by reaction with hydrogen peroxide, characterised in that the reaction is conducted in the presence of an effective quantity of zeolite of MFI structure, based on silicon oxide and germanium oxide and having, after calcination, the following formula (II):

$$Si_{(96-x)} \cdot Ge_x O_{192} \quad (II)$$

in which:

x is between 0.1 and 36, approximately.

**2.** Process according to claim 1, characterised in that it is applied to phenols and phenol ethers of formula (I) in which $R_5$ denotes a hydrogen atom, a methyl group or an ethyl group, and $R_6$ denotes a hydrogen atom, a methyl, ethyl or tert-butyl group or a methoxy or ethoxy group.

**3.** Process according to either of claims 1 and 2, characterised in that it is applied to phenols and phenol ethers chosen from phenol, anisole, ortho-cresol, meta-cresol, para-cresol, 4-tert-butylphenol, 2-methoxyphenol and 4-methoxyphenol.

**4.** Process according to one of claims 1 to 3, characterised in that the germanozeosilite contains between 0.01 and 1.4 % of fluorine by weight after calcination.

**5.** Process according to one of claims 1 to 4, characterised in that the germanozeosilite employed is obtained by a process comprising the following steps:

(i) preparation of a reaction mixture in an aqueous medium containing at least one source of silicon with a degree of oxidation of +4, a source of germanium with a degree of oxidation of +4 and a structuring agent,
(ii) crystallisation of this reaction mixture by heating and recovery of the crystallised precipitate, and
(iii) calcination of the latter at a temperature above 450°C to remove the structuring agent occluded in the channels.

6. Process according to claim 5, characterised in that during the preparation of the germanozeosilite the molar ratio Ge/(Si + Ge) in the reaction mixture is between 0.001 and 0.80 and preferably between 0.01 and 0.70 when the pH is higher than 12.

7. Process according to claim 5, characterised in that during the preparation of the germanozeosilite the molar ratio Ge/(Si + Ge) in the reaction mixture is between 0.001 and 0.75 and preferably between 0.002 and 0.60 when the pH is below or equal to 12.

8. Process according to either of claims 5 and 7, characterised in that during the preparation of the germanozeosilite the molar ratio F/(Si + Ge) in the reaction mixture is between 0.06 and 2 in the case of a pH below or equal to 12.

9. Process according to either of claims 5 and 6, characterised in that during the preparation of the germanozeosilite the molar ratio $H_2O$/(Si + Ge) in the reaction mixture is between 4 and 400 and preferably between 10 and 200, in the case of a pH higher than 12.

10. Process according to one of claims 5, ? and 8, characterised in that during the preparation of the germanozeosilite the molar ratio $H_2O$/(Si + Ge) in the reaction mixture is between 4 and 400 and preferably between 20 and 200, in the case of a pH below or equal to 12.

11. Process according to one of claims 5, 6 and 9, characterised in that during the preparation of the germanozeosilite the molar ratio structuring agent/(Si + Ge) is between 0.002 and 4 and preferably between 0.06 and 2 in the case of a pH higher than 12.

12. Process according to one of claims 5, 7, 8 and 10, characterised in that during the preparation of the germanozeosilite the molar ratio structuring agent/(Si + Ge) is between 0.002 and 4 and preferably between 0.06 and 1 in the case of a pH below or equal to 12.

13. Process according to one of claims 1 to 12, characterised in that the molar ratio phenol/hydrogen peroxide is from 25/1 to 3/1 and preferably from 20/1 to 4/1.

14. Process according to one of claims 1 to 13, carried out noncontinuously, characterised in that the catalyst represents from 0.1 % to 20 % and preferably from 0.5 % to 10 % by weight relative to the weight of the compound of formula (I) introduced.

15. Process according to one of claims 1 to 13, characterised in that the process is carried out continuously in a fixed catalyst bed.

16. Process according to one of claims 1 to 15, characterised in that hydrogen peroxide is employed in the form of an aqueous solution.

17. Process according to one of claims 1 to 15, characterised in that hydrogen peroxide is employed in the form of an organic solution.

18. Process according to one of claims 1 to 17, characterised in that the hydroxylation reaction is carried out in a solvent for the compound of formula (I), preferably miscible or partially miscible with water, such as water, an alcohol, a ketone, a nitrile, a carboxylic acid, an ester, an ether or a polar aprotic solvent.

19. Process according to one of claims 1 to 18, characterised in that the hydroxylation reaction is conducted at a temperature of between 45°C and 160°C.